# EUROPEAN PATENT SPECIFICATION

(11) **EP 2 247 624 B1**
(45) Date of publication and mention of the grant of the patent: **08.07.2020**
(21) Application number: 09712768.2
(22) Date of filing: 18.02.2009
(51) Int. Cl.: C08B 30/14, A61Q 1/10, A61K 8/73, A61K 8/02, A23K 20/163, A23P 10/40, A23L 29/212, A23L 27/00

(54) **PREGELATINIZED STARCHES AS CARRIER MATERIALS FOR LIQUID COMPONENTS**
VORGELATINISIERTE STÄRKEN ALS TRÄGERMASSEN FÜR FLÜSSIGE KOMPONENTEN
AMIDONS PRÉGÉLATINISÉS EN TANT QUE MATÉRIAUX DE SUPPORT POUR COMPOSANTS LIQUIDES

(30) Priority: 22.02.2008 EP 08003275
(43) Date of publication of application: 10.11.2010
(73) Proprietor: Cargill, Incorporated, Wayzata, MN 55391 (US)
(72) Inventor: VEELAERT, Sarah, B-1980 Eppegem (BE)
(74) Representative: Elseviers, Myriam
(86) International application number: PCT/EP2009/001160
(87) International publication number: WO 2009/103514

(56) References cited:
- US-A- 4 810 517
- US-A- 4 812 445

## Description

### TECHNICAL FIELD

The present invention concerns a powdered liquid-loaded starch material comprising a solid carrier material consisting of a pregelatinized, non-granular starch material consisting of flake-shaped starch particles, and one or more liquid components absorbed into and/or onto said solid carrier material. The present invention further concerns processes for preparing said powdered liquid-loaded starch material. In addition, the present invention relates to the use of said powdered liquid-loaded starch material in food and animal feed products, pharmaceuticals, nutraceuticals, agrochemicals, and cosmetic or personal care products.

### BACKGROUND OF THE INVENTION

Starch is a polysaccharide that is produced in the from of granules in most plant cells. Such starch granules consist of highly ordered crystalline regions and less organized amorphous regions. When present in this granular state, the starch is referred to as "native starch". Native starches from different plant sources vary widely in structure and composition, but all granules consist of two types of polysaccharides, amylose (normally 20-30%) and amylopectin (normally 70-80%), both of which are polymers of α-D-glucose. The native starches, however, have inherent disadvantages that would them render unsuited for many applications. Therefore, chemical or physical modification processes have been developed, which turn these undesirable properties into desirables ones.

A widely used physical modification is the pregelatinization of starch, which is the collapse or disruption of molecular orders within starch granules, manifested in irreversible changes in properties such as granular swelling (penetration of water, which results in an increased randomness in the granular structure), native crystallite melting (decrease of crystalline regions of the starch granules due to the penetration of water), loss of birefringence, and starch solubilization. Such pregelatinized starches are substantially soluble (swelling) in cold water without cooking and develop viscosity immediately (instant starches), in contrast to native starches.

Pregelatinized starches are typically prepared by thermal, chemical, or mechanical processes. The particular process employed strongly affects the physical properties of the pregelatinized starches, in particular wettability, dispersibility and peak viscosity in cold water. Thermal processes are widely used as heat causes the conversion of crystalline regions into amorphous region, thereby promoting the penetration of water and swelling of the granules. Typical thermal processes to effect gelatinization include spray-drying, roll-drying or drum-drying, extrusion, and other heating/drying processes.

Depending on the method used and the specific process parameters employed, the produced pregelatinized starches may have lost or maintained their granular structure. The non-granular pregelatinized starches, typically prepared by roll-drying, drum-drying, extrusion and, in some cases, spray-drying, are widely used in various technical fields (see, e.g., U.S. patents Nos. 3,607,394 and 5,131,953). For some applications, however, granular pregelatinized starches are preferentially used because the intact granular structure imparts certain properties, such as improved texture. These granular pregelatinized starches may be prepared by, for example, specific spray-drying processes, which cause swelling and pregelatinization while preventing destruction of the granule shape, or heating in aqueous organic solvents, such as alcohol-water mixtures, followed by drying (see, e.g., U.S. patents Nos. 4,465,702 and 5,037,929).

Pregelatinized starches are widely used in various technical fields to alter the viscosity or texture of a given product without requiring heating. For this reason, for example, numerous food products contain pregelatinized starches. Another important field of application is the pharmaceutical industry, where pregelatinized starches are traditionally used as a binder, filler or disintegrant, and to enhance drug stability and control release rates in modified-delivery dosage forms.

Recently, it was reported that pregelatinized starches have the potential to act as carriers for the retention and protection of volatile aroma compounds (Boutboul et al, Carbohydrate Polymers 47 (2002): 73-82). Boutboul *et al.* studied the physical characteristics (particle size, specific surface area, and particle shape) of different starches, including pregelatinized starches, and correlated these physical characteristics with the ability of the starch particles to retain volatile aroma compounds. Two different pregelatinized starches were used in this study, a pregelatinized standard corn starch and a pregelatinized (low-amylose) waxy corn starch, both produced from the respective native starches by drum-drying, which had non-granular, heterogeneous shapes and particle sizes of 11-69 µm and 10-64 µm and specific surface areas of 0.51 m²/g and 0.54 m²/g, respectively. Boutboul *et al.* found that the principal factor affecting aroma retention is the specific surface area, which depends from the shape and size of the starch particles, wherein the retention capacity increases as the specific surface increases.
US 4,810,517 relates to a leguminous Pregelatinized cold swelling starch component.

Although there are various starch-based materials available in the art, which are used as carriers or encapsulating materials for liquids, solids and volatile substances, there is still a need in the industry for an additional starch material having a high loading capacity for liquid components, which can be prepared in a simple and cost-efficient manner.

### SUMMARY OF THE INVENTION

The present invention provides a powdered liquid-loaded starch material comprising a solid carrier material consisting of a pregelatinized, non-granular starch material consisting of flake-shaped starch particles, which have a size distribution in the range of between 100 µm and 375 µm, preferably in the range of between 125 µm and 350 µm, for at least 50% by weight, preferably 80% by weight of the starch particles, and a BET specific surface of less than or equal to 0.5 m²/g, preferably less than or equal to 0.4 m²/g, and one or more liquid components absorbed into and/or onto said solid carrier material.

The powdered liquid-loaded starch material is prepared by loading a liquid component onto the pregelatinized, non-granular starch material wherein the loading is effected by applying, in particular spraying, one or more liquid components to the starch material under agitation.

In addition, the present invention relates to the use of the powdered liquid-loaded starch material as defined herein in food and animal feed products, pharmaceuticals, nutraceuticals, agrochemicals, and cosmetic and personal care products.

The present invention will now be further described by reference to the following detailed description of the present invention and the examples.

### DETAILED DESCRIPTION

The present invention is based on the unexpected finding that a pregelatinized, non-granular starch material, which consists of flake-shaped starch particles having a particle size of 100 µm to 375 µm for at least 50% by weight of the flake-shaped starch particles, have novel and superior functionalities, i.e. an excellent loading capacity for liquid components, compared to conventionally spray-dried pregelatinized starches and yet provide a powdered liquid-loaded starch material. It is known that small particles sizes and/or irregular particle shapes correlate with a high specific surface area, which in turn correlates with a high loading capacity. For that reason, small particles with a high specific surface area are normally and preferentially used for the purpose of capturing or encapsulating volatile or liquid substances. The flake-shaped starch particles are relatively large particles, and the specific surface area is as low as 0.5 m²/g or less, or 0.4 m²/g or less, or even less than 0.3 m²/g. It was therefore surprising to find that the pregelatinized, non-granular starch material exhibits a high loading capacity for liquid components like oils, and provide a powdered liquid-loaded starch material.

The term "pregelatinized starch", when used herein, means a starch that has been chemically and/or mechanically and/or thermally treated in the presence of water to decrease the number and size of crystalline regions and increase the randomness in the general structure, and has been subsequently dried. Typically, the structural changes induced by gelatinization are manifested in the loss of birefringence and/or Maltese crosses in polarized light. The pregelatinized starches may or may not have lost their granular structure and are substantially soluble in cold water without cooking. In accordance with the present invention, "pregelatinized starches" may also be chemically modified to impart desirable properties, such as flowability, hydrophobicity and the like. Preferably, the pregelatinized starch used in the present invention is not chemically modified. Furthermore, the term "pregelatinized starch" may also include partially pregelatinized starch (PPS), which contains soluble (gelatinized) and insoluble fractions. Preferably, the pregelatinized starch used in the present invention is completely or predominantly pregelatinized, i.e. with less than 10%, preferably less than 5%, in particular less than 2% or 1% by weight, of crystalline regions.

In accordance with the present invention, the term "chemically modified" or "chemical modification" includes, but is not limited to, crosslinked starches, starches modified with blocking groups to inhibit retrogradation, starches modified by the addition of lipophilic groups, acetylated starches, hydroxyethylated and hydroxypropylated starches, inorganically esterified starches, cationic, anionic and oxidized starches, zwitterionic starches, starches modified by enzymes, and combinations thereof.

Suitable pregelatinized starches for use herein can be derived from any native source, wherein native relates to the fact that said starch is found in nature. Typical sources for the starches are cereals, tubers, roots, legumes, fruit starches and hybrid starches. Suitable sources include, but are not limited to, corn, pea, potato, sweet potato, sorghum, banana, barley, wheat, rice, sago, amaranth, tapioca, arrowroot, canna, and low amylose (containing no more than about 10% by weight amylose, preferably no more than 5%) or high amylose (containing at least about 40% by weight amylose) varieties thereof. Also suitable are starches derived from a genetically modified starch crop. A preferred starch for use herein has an amylose content below 40%, including waxy corn starch with less than 1% amylose content. Particularly preferred starches include rice, wheat, tapioca, corn, and potato starches, in particular corn (maize) starch.

A "non-granular starch material", as used herein, refers to a starch material consisting of particles that do not have a granular shape. A "granular shape" is intended to mean a roughly spheroid or ellipsoid shape and includes spherical particles that have indentations in one or more portions thereof, such as the spherical starch particles produced by a conventional spray-drying process. A "flake-shaped" starch particle, when used herein, is a particle that has lost its granular structure and has a heterogeneous shape in the form of irregular flat or thick plates or sheets. Typically, roll-drying or drum-drying processes generate such flake-shaped starch particles.

The pregelatinized, non-granular starch material is characterized by a size distribution of the flake-shaped starch particles such that at least 50% by weight of the starch particles have a particle size of between 100 µm and 375 µm, as determined by a sieve analysis. Preferably, at least 80%, more preferably at least 90%, yet more preferably at least 95%, and most preferably 100% by weight of the starch particles have a particle size of between 100 µm and 375 µm. It is further preferred that the particle size for at least 80%, 90%, 95% or 100% by weight of the starch particles is in the range of 125 µm and 350 µm, more preferably between 125 µm and 325 µm, and most preferably between 125 µm and 300 µm.

The BET specific surface area of the pregelatinized, non-granular starch material is typically not higher than 0.5 m²/g, preferably less than or equal to 0.4 m²/g, and more preferably less than or equal to 0.3 m²/g. A particularly preferred pregelatinized, non-granular starch material has a particle size of 100 µm to 375 µm for at least 50% by weight, preferably 80% by weight, of the starch particles, and a BET specific surface area of less than or equal to 0.5 m²/g, preferably less than or equal to 0.4 m²/g.

According to the present invention, the pregelatinized, non-granular starch material may include minor amounts of one or more additives, preferably in a total amount of no more than 10% by weight, more preferably no more than 5% by weight, most preferably 0% to 1% by weight, based on the total weight of the starch particles.

These optionally present additives are added to the starting starch slurry or paste used for preparing the pregelatinized, non-granular starch material of the present invention. Examples of additive include, but are not limited to, processing aids, such as agents for enhancing the formation of bubbles, surfactants and emulsifiers, and other ingredient, such as salts, sugars, fat, gums and hydrocolloids. Although not preferred, the additives included in the pregelatinized, non-granular starch material may also be substances that have been added to the formed pregelatinized, non-granular starch material to provide it with desirable properties. An example thereof is a surface modifying agent, which changes the absorption properties of the starch to improve, for example, the absorption of hydrophobic ingredients like oils and fats.

Preferably the pregelatinized, non-granular starch material consisting of flake-shaped starch particles as described hereinabove is produced by a roll-drying or drum-drying process. Roll-drying as well as drum-drying involve the heating of an aqueous starch slurry or paste to gelatinize the starch and to instantaneously remove the moisture. The aqueous starch slurry or paste may be first heated and subsequently dried or, more preferably, the starch may be simultaneously gelatinized by heating and dried using a commercially available drum-dryer or roll-dryer apparatus. As used herein, the term "roll-drying" refers to a process where an aqueous starch slurry or paste is cooked or partially cooked and passed on heated rolls (sometimes also referred to as "drums") for drying or, preferably, a process where the aqueous starch slurry or paste is simultaneously cooked and dried on heated rolls. The term "drum-drying", when used herein, refers to a process very similar to the roll-drying process, except that a thicker coating of the starch slurry or paste is applied to heated drums.

A preferred process for preparing the pregelatinized, non-granular starch material described hereinabove starts with mixing starch (generally in the form of a starch powder) and water to prepare an aqueous starch slurry or paste having a certain solids content. A "starch slurry or paste" may also include high-viscosity starch preparations, such as a moist filter cake. Suitable starches are as defined above. The starch content typically ranges from 20 to 45% by weight, in particular from 25 to 40% by weight, and especially from 32 to 40% by weight.

The prepared aqueous starch slurry or cake is then applied onto heated, rotating rolls or drums of a roll-dryer or drum-dryer, conveniently by means of application drums or feed rolls, to simultaneously gelatinize and dry the aqueous starch slurry or paste. After one rotation, the obtained dried starch film is removed from the rolls or drums by a scrapping mechanism, such as a knife blade, to obtain a starch material, which is then subjected to grinding or milling, for example in a rotor beater mill or cutting mill. Finally, the ground (milled) starch material is sieved using one or several sieves of different mesh sizes, as known in the art, to obtain the desired sieve fraction of the pregelatinized, non-granular starch material.

Suitable roll-dryers and drum-driers for preparing the pregelatinized, non-granular starch material of the present invention are commercially available, for example from GMF-Gouda (The Netherlands). Typically, they are designed as indirect dryers, where heat is transferred by pressurized stream to the inside (metal) drum wall, which in turn transfers the heat to the aqueous starch slurry or paste on the other side of the wall. While the basic construction is relatively simple, there are numerous configurations commercially available, which differ in the arrangement and number of drums and feed rolls, the type of scrapping mechanism, etc.

Factors, such as the composition of the aqueous slurry or paste, the roll or drum temperature, and the drum or roll speed (which determines the residence time), will have an effect on the physical and chemical properties of the final pregelatinized, non-granular starch material. A person skilled in the art of roll-drying or drum-drying is familiar with these parameters and knows how to set or adjust these parameters to obtain a pregelatinized starch material having desirable properties. For example, different types of starches are known to have varying gelatinization temperatures and thus one or more of the above parameters may be adjusted and optimized to achieve a satisfactory result. Such optimizations are well within the normal capabilities of a person skilled in the art of drum-dried or roll-dried pregelatinized starches.

The rolls or drums are typically heated to have a surface temperature in the range of 120 to 200 °C, in particular in the range of 140 to 190°, and especially in the range of 150 to 180°C. The rolls or drums are normally operated at a speed or rotation rate of 5 to 18 rpm, preferably of 5 to 15 rpm, and more preferably of 8 to 13 rpm.

One or more additional constituents (additives) may be admixed to the aqueous starch slurry or paste including, but not limited to, processing aids, such as bubble-forming agents, surfactants and emulsifiers, and other substances, such as salts, sugars, fat, gums, and hydrocolloids to improve certain properties. For example, the starch slurry or paste applied to the heated rolls or drums gets transformed into a continuous phase of melted starch that includes variable amounts of air bubbles. In order to obtain a pregelatinized starch material with an increased absorption capacity, conditions might be chosen to result in a relatively low bulk density, for example, by adding specific processing aids to the aqueous starch slurry or paste to increase formation of bubbles.

The additional constituents, if present, are added in small amounts, normally in amounts such that the additional constituents make up no more than 10% by weight, preferably no more than 5% by weight, more preferably no more than 1% by weight, of the total weight of the final roll-dried or drum-dried starch particles. In other embodiments of the present invention, 0% of these additional ingredients are added, and the aqueous starch slurry or paste therefore consists exclusively of starch and water.

Furthermore, it is also within the scope of the present invention, although not especially preferred, that the obtained roll-dried or drum-dried, pregelatinized, non-granular starch material is additionally treated with a surface modifying agent to change the absorption properties of the starch. A hydrophobic agent, for example, will further improve the absorption capacity for hydrophobic liquid components, like oils and fats.

The powdered liquid-loaded starch material comprises beyond the solid carrier material as defined hereinabove one or more liquid components. The one or more liquid components are buried within, supported on, captured by, bound to, and/or absorbed into and/or onto the carrier material, which provides a matrix for the liquid components. It will be understood, that the term "comprise", as used in the present description, is meant to encompass not only the meanings "include", "contain" or "comprehend" but also the meaning of "consisting (exclusively) of".

A "liquid component", as used herein, refers to any matter that is present in liquid form and includes, for example, mixtures of different liquids and solutions or suspensions of one or more substances. Liquid ingredients that can be loaded include, but are not limited to, flavour compounds, aromas, fragrances, plant derived extracts, emulsifiers, colours, oils and fats, in particular those oils and fats that are usable as food ingredients or additives, such as omega-3 rich oils, salad and fish oils, essential oils and lecithin, other nutrients, such as carotenoids and vitamins like vitamins A and E, and organic acids antioxidants, pharmaceutically active ingredients, as well as oleoresins, blood, alcoholic beverages, insect repellents, insecticides, and herbicides. Furthermore, also solutions of specific substances or ingredients, such as biologically active compounds like microorganisms or enzymes, are suitable for use herein, wherein the solvent, if desired, can be removed after loading by a drying step. Preferred liquid ingredients are alcohols, acetones, ketones, aldehydes, oils and fats. Particularly suitable for use herein are hydrophobic liquids, in particular any type of oil and fat, essential oils, oleoresins, plant derived extracts, flavour and fragrance blends in carrier solvents like alcohol, propylene glycol, water or vegetable oils, lecithin and polyunsaturated fatty acids.

In a preferred embodiment of the present invention, the powdered liquid-loaded starch material comprises no less than 20% by weight of the one or more liquid components, based on the total weight of the liquid-loaded starch particles. More preferably, the liquid components account for at least 25% or 30%, more preferably for at least 35%, and most preferably for at least 40% by weight, of the powdered liquid-loaded starch particles.

In a further aspect, the present invention relates to a process for preparing the powdered liquid-loaded starch material as described hereinabove by applying one or more liquid components to the pregelatinized, non-granular starch material consisting of flake-shaped starch particles as described hereinabove under agitation.

For loading the pregelatinized, non-granular starch material with one or more liquid components, the starch material may be placed in a vessel supporting mechanical mixing and preferable capable of being sealed. Suitable mixing devices are, for example, a paddle mixer, a ribbon blender, a V-blender, or a plough blade mixer. The one or more liquid components are then supplied, for example poured, pumped or, preferably, sprayed via a nozzle, into the vessel and applied onto the agitated starch material. Spraying via a nozzle is advantageously used because the nozzle leads to the formation of small droplets that are more easily absorbed by the starch carrier material. Loading from the gas phase or under supercritical conditions is also possible. The mixing is continued until an even distribution of the liquid material into and/or onto the solid carrier is obtained. The time required for spraying or pumping is dependent upon the addition level of the liquid components onto the pregelatinized starch material and the time required to ensure complete absorption to form a free flowing powder.

Another suitable method for loading one or more liquid components onto the pregelatinized, non-granular starch material may be a fluidized-bed loading process. In such a process, the carrier, i.e. the pregelatinized, non-granular starch material is fluidized by forcing air or another gas upward through a bed of starch particles. The liquid components are then sprayed via a nozzle onto the fluidized starch particles to yield a liquid-loaded starch material of evenly loaded starch particles.

A further suitable loading method for use herein comprises the steps of suspending the pregelatinized, non-granular starch carrier material in the liquid components, followed by separating the powdered liquid-loaded starch material from the liquid components by conventional separation methods, such as filtration or centrifugation.

Depending on the type of liquid component to be loaded, the liquid component may be heated or cooled. In case of high viscous liquid components, for example, it might be favourable to heat the liquid components to decrease the viscosity and facilitate the loading process. In case of temperature-sensitive liquid components, cooling might be desired or required, such as for solutions of heat-sensitive pharmaceutical active substances. Means for effecting cooling or heating, such as a cooled or heated blender, are well-known to a person skilled in the art.

In accordance with the present invention, the pregelatinized, non-granular starch material used as a carrier material may be pre-treated before loading with an inert gas to remove, for instance, oxygen. It can also be vacuum-treated before loading to increase the absorption capacity. Further, when sensitive liquids are to be loaded, the loading operation might be carried out under an inert gas atmosphere, for example under a nitrogen atmosphere to protect against loss of quality by oxidation.

After having loaded the pregelatinized, non-granular starch material with one or more liquid components, further processing steps may optionally follow. For example, flowing or anti-caking agents may be added to the liquid-loaded starch material, such as tricalcium phosphate, silica, silicates and/or stearates, to increase flowability. The powdered liquid-loaded starch material of the present invention may also be provided with a coat and/or further encapsulated by any suitable encapsulating or coating materials, such as maltodextrins, starches, modified starches, dextrins, oils, fats, waxes, hydrocolloids, proteins, as known in the art.

The pregelatinized, non-granular starch material of flake-shaped starch particles is used as a carrier material for liquid components to protect, store, stabilize, and/or control the release property of the same. Furthermore, when bound to the starch carrier material, the liquid components are easier to handle, store and formulate.

The powdered liquid-loaded starch material of the present invention may be incorporated into numerous different formulations, such as powders, granules, tablets, capsules, lozenges, pastes, gels, creams, salves, ointments, lotions, and the like. Preferred end-use applications of the powdered liquid-loaded starch material of the present invention include, but are not limited to, food and animal feed products, pharmaceuticals, nutraceuticals, agrochemicals, such as herbicides, pesticides, and fertilizers, and cosmetic and personal care products, such as dry hair care products, shampoos, conditioners, antiperspirants, deodorants, mouthwashes, soaps, cosmetic creams, and the like.

In a preferred embodiment of the present invention, the powdered liquid-loaded starch material of the present invention is used in food products including, but not limited to, dry soup mixes, instant drinks and soups, cakes and dry dessert mixes, spices, seasonings, toppings, batters, milk replacements, non-dairy creams, grated or powdered cheeses, and flavoured teas.

The present invention will now be further illustrated and explained by reference to the examples given below. The methods to assess the physical characteristics of the pregelatinized, non-granular starch material provided in the examples were as follows.

### (1) Particle size distribution

The particle size distribution of starch samples was determined by a sieve analysis using sieves with different openings. The respective sieve fractions on the sieves were weighted and divided by the total weight of the starch sample to give a percentage retained on each sieve.

### (2) Particle shape

The particle shape of starch samples was observed by scanning electronic microscopy at magnifications of 100 to 750 x, as known in the art.

### (3) BET specific surface area

The specific surface area of starch samples was measured by nitrogen absorption in a Gemini II 2370 Surface Area Analyzer (Micromeritrics NV/SA, Brussels, Belgium). The multi-point (11 points by convention) BET-method (Bruauner, Emmett and Teller, J. Am. Chem. Soc. 60:309-319 (1938)) was used to determine the total available surface area (BET specific surface area) (m²/g).

### (4) Oil absorption capacity

The oil absorption capacity was measured by centrifuging a given amount of a starch sample in oil dispersion, removing the oil that had not bound to the starch, subjecting the remaining oil-loaded starch sample to high centrifugal forces and determining the amount of oil, which remained bound to the starch sample by assessing the weight of the obtained centrifuged starch.

### Sunflower oil absorption

25 g (W₀) of a starch sample was weighted and 25 g of sunflower oil (Vandemoortele, Belgium) was added and thoroughly mixed with a spoon for 2 min to give an oil-starch mixture. In case of a too high viscosity, an additional amount of oil was added. A 750 ml round bucket centrifuge bottle was filled with about 360 g native potato starch and a folded filter paper (150 mm diameter, Machery-Nagel MN 614) was unfolded and placed on top of the potato starch (in a small hole, to ensure that the filter paper will stay in position during the subsequent centrifugation). The prepared oil-starch mixture was then poured onto the filter paper, followed by centrifugation at 3434 x g for 10 min in a Heraeus Multifuge 3S centrifuge. After completion of the centrifugation, the filter paper with the starch-oil sample was withdrawn from the centrifuge bottle, and the starch-oil sample remaining on the filter was carefully removed and the weight Wₛ was measured. The oil absorbed by the sample is calculated as Wₛ-W₀ and the oil absorption capacity (%) is expressed as (Wₛ-W₀)/W₀ x 100%.

### Orange flavour absorption

The same procedure as described above for sunflower oil was used, except that the sample was centrifuged at 214.66 x g for 3 min. The used orange oil was provided by Cargill Flavour Systems Uden (CAS 8028-48-6).

### Example 1

A roll-dried regular corn starch (C*12001, available from Cargill) was sieved over 125 µm and 315 µm sieves and the sunflower oil absorption capacity was measured using the above-defined measuring method on the fraction with a particle size between 125 µm and 315 µm. In addition, the BET specific surface area of this fraction was measured with the BET method described above. The oil absorption capacity for sunflower oil was 25% and the BET specific surface area was 0.24 m²/g (Table 1).

### Example 2

A roll-dried POCl₃ crosslinked corn starch (C*12930, available from Cargill) was milled over a RETSCH SR300 rotor beater mill and sieved to remove the smaller particles below 100 µm. The resulting material is characterized by having 51% by weight of the material in the particle size range of between 100 µm and 375 µm. The oil absorption capacity was measured for sunflower oil and orange flavour with the method described above. In addition, the BET specific surface area was measured with the BET method described above. The oil absorption capacity was 21% and 28% for sunflower oil and orange flavour, respectively, and the BET specific surface area was 0.26 m²/g (Table 1).

### Example 3

Regular corn starch (C*03401, available from Cargill) was slurried in water at a concentration of 20 Be. The slurry was roll-dried on a pilot roll-dryer from GMF Gouda, Type E 5/5, with a roller (drum) of 500 x 500 mm, operated at 11 rpm and a steam pressure of 7 bar. The maximum surface temperature of the drum was 165°C. The gap clearance was 1.6 mm (measured at room temperature). The samples were sieved over 125 µm and 350 µm sieves and the absorption of sunflower oil was measured on the fraction with a particle size between 125 µm and 350 µm using the measuring method described above. In addition, the BET specific surface area for this fraction was measured with the BET method described above. The oil absorption capacity for sunflower oil was 31% and the BET specific surface area was 0.26 m²/g (Table 1).

### Example 4

Starch sodium octenyl succinate (C*06369, available form Cargill) was roll-dried as described in Example 3, except that the drum speed was 13 rpm, the steam pressure was 8.5 bar, and the maximum drum temperature was 173°C. The starch film removed from the drum was milled and sieved. The sieve fraction with a particle size of between 125 µm and 325 µm was used to measure the absorption capacity for sunflower oil and the BET specific surface area using the measuring methods described above. The oil absorption capacity for sunflower oil was 37% and the BET specific surface area was 0.23 m²/g (Table 1).

### Example 5 (Comparative Example)

A roll-dried regular corn starch (C*12001, available from Cargill) was sieved over a 50 µm sieve and the sunflower oil absorption capacity was measured using the above-defined measuring method on the fraction with a particle size below 50 µm. In addition, the BET specific surface area of this fraction was measured with the BET method described above. The oil absorption capacity for sunflower oil was 15% and the BET specific surface area was 0.52 m²/g (Table 1).

**Table 1. Physical characteristics and oil retention capacities of the pregelatinized, non-granular starch materials of Examples 1 to 4 and Comparative Example 5.**

| Example/ Comparative Example | Particle shape | Particle size (µm) | BET specific surface area (m²/g) | Oil absorption capacity (%) |
|---|---|---|---|---|
| Ex. 1 | Flakes | 125-315^{a} | 0.24 | 25^{c} |
| Ex. 2 | Flakes | 100-375^{b} | 0.26 | 21^{c}, 28^{d} |
| Ex. 3 | Flakes | 125-350^{a} | 0.26 | 31^{c} |
| Ex. 4 | Flakes | 125-325^{a} | 0.23 | 37^{c} |
| Comp. Ex. 5 | Flakes | < 50^{a} | 0.52 | 15^{c} |

| | | | | |
|---|---|---|---|---|
| ^{a} For 100% of the particles ^{b} For 51% of the particles ^{c} Sunflower oil ^{d} Orange flavour | | | | |

## Claims

1. A powdered liquid-loaded starch material, comprising a solid carrier material consisting of a pregelatinized, non-granular starch material consisting of flake-shaped starch particles, wherein the size distribution of the starch particles is such that at least 50% by weight of the starch particles have a particle size of between 100 µm and 375 µm, as measured by the method mentioned in the description, and wherein the BET specific surface area is less than or equal to 0.5 m²/g, as measured by the method mentioned in the description, and one or more liquid components are absorbed into and/or onto said solid carrier material

2. The powdered liquid-loaded starch material according to claim 1 wherein at least 80% by weight of the starch particles have a particle size of between 100 µm and 375 µm.

3. The liquid-loaded starch material of claim 1 or 2, wherein the one or more liquid components absorbed into and/or onto the solid carrier material constitute at least 20% by weight of the total weight of the liquid-loaded starch particles.

4. A process for preparing a powdered liquid-loaded starch material according to anyone of claims 1 to 3, comprising the steps of:
(1) providing a pregelatinized, non-granular starch material consisting of flake-shaped starch particles,
(2) loading one or more liquid components into and/or onto the starch material provided in step (1) by applying one or more liquid components to the starch material under agitation.

5. A process according to claim 4 wherein the pregelatinized, non-granular starch material consisting of flake-shaped starch particles is provided by the steps of:
a) mixing starch and water to prepare an aqueous starch slurry or paste,
b) applying the aqueous starch slurry or paste onto heated, rotating rolls or drums to simultaneously gelatinize and dry the aqueous starch slurry or paste to obtain a dried starch film,
c) removing the dried starch film from the rolls or drums,
d) grinding the removed dried starch film to obtain a ground starch material, and
e) sieving the ground starch material to obtain the pregelatinized, non-granular starch material consisting of flake-shaped starch particles.

6. The process of claim 4 or 5, wherein flowing agents and/or anti-caking agents are added to the liquid-loaded starch particles.

7. Use of a powdered liquid-loaded starch material according to anyone of claims 1 to 3 in food and animal feed products, pharmaceuticals, nutraceuticals, agrochemicals, and cosmetic or personal care products.

## Patentansprüche

1. Pulverförmiges flüssigkeitsbeladenes Stärkematerial, umfassend ein festes Trägermaterial, das aus einem vorgelierten, nicht körnigen Stärkematerial, bestehend aus flockenförmigen Stärketeilchen, besteht, wobei die Größenverteilung der Stärketeilchen so ist, dass mindestens 50 Gewichts-% der Stärketeilchen eine Teilchengröße zwischen 100 µm und 375 µm aufweisen, wie nach dem in der Beschreibung erwähnten Verfahren gemessen, und wobei die BET-spezifische Oberfläche weniger als oder gleich 0,5 m²/g beträgt, wie nach dem in der Beschreibung erwähnten Verfahren gemessen, und ein oder mehrere flüssige Bestandteile in und/oder auf dem festen Trägermaterial absorbiert sind.

2. Pulverförmiges flüssigkeitsbeladenes Stärkematerial nach Anspruch 1, wobei mindestens 80 Gewichts-% der Stärketeilchen eine Teilchengröße zwischen 100 µm und 375 µm aufweisen.

3. Pulverförmiges flüssigkeitsbeladenes Stärkematerial nach Anspruch 1 oder 2, wobei der eine oder die mehreren in und/oder auf dem festen Trägermaterial absorbierten flüssigen Bestandteile mindestens 20 Gewichts-% des Gesamtgewichts der flüssigkeitsbeladenen Stärketeilchen ausmachen.

4. Verfahren zum Herstellen eines pulverförmigen flüssigkeitsbeladenen Stärkematerials nach einem der Ansprüche 1 bis 3, das die folgenden Schritte umfasst:
(1) Bereitstellen eines vorgelierten, nicht körnigen Stärkematerials, bestehend aus flockenförmigen Stärketeilchen,
(2) Laden einer oder mehrerer flüssiger Bestandteile in und/oder auf das in Schritt (1) bereitgestellte Stärkematerial durch Aufbringen eines oder mehrerer flüssiger Bestandteile auf das Stärkematerial unter Rühren.

5. Verfahren nach Anspruch 4, wobei das vorgelierte, nicht körnige Stärkematerial, bestehend aus flockenförmigen Stärketeilchen, durch die folgenden Schritte bereitgestellt wird:
a) Mischen von Stärke und Wasser, um eine wässrige Stärkeaufschlämmung oder -paste herzustellen,
b) Aufbringen der wässrigen Stärkeaufschlämmung oder -paste auf erwärmte, sich drehende Walzen oder Trommeln, um die wässrige Stärkeaufschlämmung oder - paste gleichzeitig zu gelieren und zu trocknen, um einen getrockneten Stärkefilm zu erhalten,
c) Entfernen des getrockneten Stärkefilms von den Walzen oder Trommeln,
d) Mahlen des entfernten getrockneten Stärkefilms, um ein gemahlenes Stärkematerial zu erhalten, und
e) Sieben des gemahlenen Stärkematerials, um das vorgelierte, nicht körnige Stärkematerial, bestehend aus flockenförmigen Stärketeilchen, zu erhalten.

6. Verfahren nach Anspruch 4 oder 5, wobei den flüssigkeitsbeladenen Stärketeilchen Verlaufmittel und/oder Antiklumpmittel zugesetzt werden.

7. Verwendung eines pulverförmigen flüssigkeitsbeladenen Stärkematerials nach einem der Ansprüche 1 bis 3 in Lebensmitteln und Tierfutterprodukten, Pharmazeutika, Nutrazeutika, Agrochemikalien und Kosmetik- oder Körperpflegeprodukten.

## Revendications

1. Matériau d'amidon pulvérulent chargé de liquide, comprenant un matériau de support solide consistant en un matériau d'amidon prégélatinisé non granulaire consistant en des particules d'amidon en forme de flocon, dans lequel la distribution granulométrique des particules d'amidon est telle qu'au moins 50% en poids des particules d'amidon présentent une taille de particule située entre 100 µm et 375 µm, telle que mesurée par le procédé mentionné dans la description, et dans lequel la surface spécifique BET est inférieure ou égale à 0,5 m²/g, telle que mesurée par le procédé mentionné dans la description, et un ou plusieurs composants liquides sont absorbés dans et/ou sur ledit matériau de support solide.

2. Matériau d'amidon pulvérulent chargé de liquide selon la revendication 1, dans lequel au moins 80 % en poids des particules d'amidon présentent une taille de particule située entre 100 µm et 375 µm.

3. Matériau d'amidon chargé de liquide selon la revendication 1 ou 2, dans lequel les un ou plusieurs composants liquides absorbés dans et/ou sur le matériau de support solide constituent au moins 20 % en poids du poids total des particules d'amidon chargées de liquide.

4. Procédé de préparation d'un matériau d'amidon pulvérulent chargé de liquide selon l'une quelconque des revendications 1 à 3, comprenant les étapes suivantes :
(1) la fourniture d'un matériau d'amidon prégélatinisé non granulaire consistant en des particules d'amidon en forme de flocon,
(2) le chargement d'un ou de plusieurs composants liquides dans et/ou sur le matériau d'amidon fourni dans l'étape (1) en appliquant un ou plusieurs composants liquides sur le matériau d'amidon sous agitation.

5. Procédé selon la revendication 4 dans lequel le matériau d'amidon prégélatinisé non granulaire consistant en des particules d'amidon en forme de flocon est fourni par les étapes suivantes :
a) le mélange d'un amidon et d'eau pour préparer une suspension ou pâte aqueuse d'amidon,
b) l'application de la suspension ou pâte aqueuse d'amidon sur des rouleaux ou tambours rotatifs chauffés pour gélatiniser et sécher simultanément la suspension ou pâte aqueuse d'amidon afin d'obtenir un film d'amidon sec,
c) le retrait du film d'amidon sec à partir des rouleaux ou tambours,
d) le broyage du film d'amidon sec retiré pour obtenir un matériau d'amidon broyé, et
e) le tamisage du matériau d'amidon broyé pour obtenir le matériau d'amidon prégélatinisé non granulaire consistant en des particules d'amidon en forme de flocons.

6. Procédé selon la revendication 4 ou 5, dans lequel des agents d'écoulement et/ou des agents antiagglomérants sont ajoutés aux particules d'amidon chargées de liquide.

7. Utilisation d'un matériau d'amidon pulvérulent chargé de liquide selon l'une quelconque des revendications 1 à 3 dans des produits alimentaires et d'alimentation pour animaux, des produits pharmaceutiques, des nutraceutiques, des produits agrochimiques, et des produits cosmétiques ou de soins personnels.
